# EUROPEAN PATENT SPECIFICATION

(11) **EP 1 057 923 B1**
(45) Date of publication and mention of the grant of the patent: **07.05.2003**
(21) Application number: 00870016.3
(22) Date of filing: 07.02.2000
(51) Int. Cl.: D06F 73/02

(54) **Apparatus for cleaning and refreshing fabrics**
Vorrichtung zum Reinigen und zum Auffrischen von Textilien
Dispositif pour nettoyer et rafraîchir les tissus

(30) Priority: 04.06.1999 US 137677 P
(43) Date of publication of application: 06.12.2000
(73) Proprietor: WHIRLPOOL CORPORATION, Benton Harbor Michigan 49022 (US)
(72) Inventor: van Hauwermeiren, Tim, 1880 Ramsdonk (BE); Bettiol, Jean-Luc Philippe, 1200 Brussels (BE); Bouvette, Marianne, 1853 Strombeek-Bever (BE); Dewaele, Joost, 8570 Vichte (BE); Pasin, Merih, 1180 Brussels (BE); Vos, Eddy, 3210 Linden (BE); Yeazell, Bruce Albert, Cincinnati, Ohio 45231 (US); Gaaloul, Sonia, 1070 Brussels (BE)
(74) Representative: Guerci, Alessandro

(56) References cited:
- DE-A- 2 357 646
- DE-A- 2 433 909
- FR-A- 1 404 191
- FR-A- 2 298 636
- GB-A- 1 414 041
- US-A- 3 576 079
- US-A- 3 601 292

## Description

### TECHNICAL FIELD

The present invention relates to apparatuses useful for cleaning and refreshing fabrics in a non-immersion cleaning process. Temperature and relative humidity are controlled in the apparatuses of this invention.

### BACKGROUND OF THE INVENTION

Certain delicate fabrics are not suitable for conventional in-home immersion cleaning processes. Home washing machines, which provide excellent cleaning results for the majority of fabrics used in today's society, can, under certain conditions, shrink or otherwise damage silk, linen, wool and other delicate fabrics. Consumers typically have their delicate fabric items "dry-cleaned". Unfortunately, dry-cleaning usually involves immersing the fabrics in various hydrocarbon and halocarbon solvents that require special handling and the solvent must be reclaimed, making the process unsuitable for in-home use. Hence, dry-cleaning has traditionally been restricted to commercial establishments making it less convenient and more costly than in-home laundering processes.

Attempts have been made to provide in-home dry-cleaning systems that combine the fabric cleaning and refreshing of in-home, immersion laundering processes with the fabric care benefits of dry-cleaning processes. One such in-home system for cleaning and refreshing garments comprises a substrate sheet containing various liquid or gelled cleaning agents, and a plastic bag. The garments are placed in the bag together with the sheet, and then tumbled in a conventional clothes dryer. In a current commercial embodiment, multiple single-use flat sheets comprising a cleaning/refreshing agent and a single multi-use plastic bag are provided in a package.

Unfortunately, such in-home processes are designed for use in a conventional clothes dryer, or the like apparatus. Such apparatuses are not always readily available, and they are often uneconomical. Moreover, in many countries clothes dryers are simply unnecessary. For example, in many warm tropical regions people do not typically own clothes dryers because their clothes can be dried year-round by hanging them outside in the sun. In the areas of the world where people do not typically own clothes dryers, products that require a heating apparatus, such as a clothes dryer, are of little or no value.

Steamer cabinets have also been utilized in the past to treat fabric articles with heavy doses of steam. Unfortunately, past steam cabinets were largely uncontrolled with respect to temperature and humidity. The cabinets were generally large appliances that were not portable. And due to the large amount of steam used a drying step is often required that puts strain on the fabrics. The drying step also requires additional time and energy, and often results in undesirable shrinkage.

DE 2357646 discloses a cloth drying and refreshing apparatus that comprises a flexible and thus foldable container, which can be arranged in-between two rigid end-portions. D1 is however silent about the possibility of incorporating a filter to the apparatus to retain malodorous / undesirable actives from the air that is exhausted outside said apparatus.

US 3.601.292 discloses a garment treating apparatus that comprises a collapsible container and a system for producing and circulating a garment treating composition inside the container. D2 Is silent about the possibility of incorporating a filter to the apparatus to retain malodorous / undesirable actives from the air that is exhausted outside said apparatus.

US 3.576.079 discloses a portable garment steaming and drying apparatus, comprising a collapsible chamber and a system for producing and circulating a garment treating composition into said chamber. D3 does not disclose nor suggest the possibility of incorporating a filter to the apparatus.

GB 1.414.041 discloses a cloth drying and refreshing apparatus that comprises a system for producing and circulating a garment treating composition into said container. D4 is however silent about the possibility of incorporating a filter to the apparatus.

DE 2433909 discloses a garment care device that comprises a fresh air filter that takes the form of a drawer built-in the device, and therefore takes wherein little space. D5 however does not disclose or even suggests that the device could be collapsible.

FR 2.298.636 discloses a portable garment steaming and drying apparatus, comprising a collapsible chamber and a system for producing and circulating a garment treating composition into said chamber. D6 does not disclose nor suggest the possibility of incorporating a filter to the apparatus.

Thus, there is a need to develop a domestic, non-immersion cleaning and refreshing process, and cleaning and refreshing compositions for use therein, which provides acceptable cleaning without the need for a tumble dryer. Moreover, there is a need for apparatuses that can regulate both temperature and relative humidity within a container during a domestic, non-immersion cleaning and refreshment process, wherein dry clean only fabrics are cleaned, dewrinkled and refreshed. The present invention provides such apparatuses for cleaning and refreshing fabrics.

### SUMMARY OF THE INVENTION

In one aspect of the present invention there is provided an apparatus for treating a fabric article comprising:
a) a collapsible or expandable container that is made from a material, wherein the material defines an interior void space having an open volume of between about 0.75 m³ and about 0.05 m³, preferably between about 0.6 m³ and about 0.1 m³, between about 0.5 m³ and about 0.2 m³, and further comprising an opening;
b) a humidity provider;
c) a heating element;
d) a vent; and
e) an air circulation device.

Wherein the container can be collapsed to at least about 50%, preferably at least about 40%, and more preferably at least about 25% of its open volume. It is an essential feature of the invention that said apparatus comprises a filter, wherein the vent (28) and the air circulation device are dimensioned in order to have a volume refreshment rate of between 0,004 s⁻¹ and 0,05 s⁻¹, preferably between 0,001 s⁻¹ and 0,035 s⁻¹. Preferably the material is a flexible material, more preferably is a coated fabric material that can withstand temperatures of about 100°C with essentially no increase in its vapor permeability or loss in mechanical properties. In one embodiment of this invention there is provided a hangar for suspending at least one fabric article within the interior void space of the container.

The apparatuses of this invention preferably comprise a temperature controller capable of changing and maintaining the air temperature within the interior void space of the container. Still, preferably, the humidity provider is a humidity controller capable of changing and maintaining the relative humidity of the air within the interior void space of the container.

The apparatuses of this invention also preferably weigh less than about 15 Kg, preferably less than about 10 Kg, and more preferably less than about 8 Kg, and even more preferably comprise an ozone source within the interior void space of the container that can be, for example an ultraviolet lamp, or even a high voltage source.

The present inventions provide benefits over the prior art in that fabric articles can be cleaned and refreshed without the need for expensive mechanical apparatuses, such as a tumble clothes dryer. Moreover, fabric refreshment processes comprising two or more steps with regard to temperature and humidity can be accomplished in the present apparatuses. Further, the present invention provides an apparatus for delivering an active ingredient, for example, perfume, to the fabrics being treated. Simultaneously, the apparatuses herein minimize the amount of vapor delivered so that the actives are not wasted. And finally, the apparatuses herein are designed to deliver only enough vapor to accomplish the fabric treatment process, without the need for additional mechanical drying.

### BRIEF DESCRIPTION OF THE DRAWINGS

While this specification concludes with claims that distinctly define the present invention, it is believed that these claims can be better understood by reference to the Detailed Description Of The Invention and the drawings, wherein:
Figure 1 is a schematic representation of a fabric treatment apparatus according to the present invention;
Figure 2 is the top view of the fabric treatment apparatus of Figure 1 after it has been collapsed;
Figure 3 is the front view of the fabric treatment apparatus of Figure 1 after it has been collapsed;
Figure 4 is a cross sectional view of the fabric treatment apparatus of Figure 3, wherein a fluid containing cannister has been added;
Figure 5 is a schematic drawing of one possible arrangement of the mechanical components of the present invention; and
Figure 6 is a lengthwise cross sectional view of a fabric treatment apparatus comprising a conduit according to the present invention.

### DETAILED DESCRIPTION OF THE INVENTION

The present invention provides apparatuses for cleaning and refreshing fabric articles in a domestic, non-immersion process. The apparatuses are suitable for use in a cleaning and refreshing method that requires at least two steps, and preferably three. The temperature and relative humidity within the fabric treatment apparatus can be manipulated and controlled to create a warm, humid environment inside the container of the fabric treatment apparatus. This controlled environment volatilizes malodor components in the manner of a "steam distillation" process, and moistens fabrics and the soils thereon. This moistening of fabrics can loosen pre-set wrinkles, and because the fabric articles are hung in the container new wrinkles do not form. Proper selection of the amount of the vapor, and specifically the amount of water used in the process and, importantly, proper venting of the container in the present manner can minimize shrinkage of the fabrics. Moreover, if the container is not vented, the volatilized malodorous materials removed from the fabrics, which are not captured by the filter, can undesirably be re-deposited thereon.

Relative humidity is a well known concept to those in the fabric care arts. As used herein, "relative humidity" means the ratio of the actual amount of water vapor in the air to the greatest amount the air can hold at the same temperature.

Temperature and relative humidity controllers are well known to those skilled in the art, as are passive and active controllers. As used herein, an "active" controller is a controller that reads an input and supplies feedback to the device being controlled and that device adjusts based on the feedback received. A "passive" controller, as used herein, is a controller that turns a device on or off, or opens or closes a device, based on a predetermined setting such as time. For example, a passive temperature controller would turn on a heating element or close a vent to increase the temperature in a given environment and after a certain period of time the heating element is turned off or the vent is opened. In contrast, an active temperature controller reads the temperature and if, for example, the temperature is too low, the power to the heating element is increased or the vent is closed to increase the temperature.

As used herein "fabric articles" is meant to encompass any and all articles of manufacture that are made at least partially of a natural or manmade fibrous material. Examples of fabric articles include, but are certainly not limited to: toys, shoes upholstery, garments, carpets, clothes hats, socks, towels, draperies, etc.

### Apparatus

The fabric care apparatuses of this invention can take a variety of forms. But it is generally preferred that the apparatuses comprise a container that substantially encloses the fabric articles being cleaned and refreshed. By "substantially encloses", it is meant that the fabric articles are enclosed in the container, but that the container can, and preferably will, include one or more vents. The container must have an opening to access the fabric articles, and preferably, there is a bar, hook or other device on which to hang the fabric articles.

The container preferably has only one wall configured like an egg shell. It has been found that the vapor, and subsequently the active ingredients, preferentially condense in the corners and along the sharp edges of a more conventional rectangular shaped cabinet. This is not to say that the methods of this invention cannot be conducted in rectangular cabinets; they can. Regardless of its shape, every container has an "open volume" which as used herein means the volume of the container when it is in use. The containers of this invention are collapsible or expandable and have a substantially reduced volume in their closed or collapsed state.

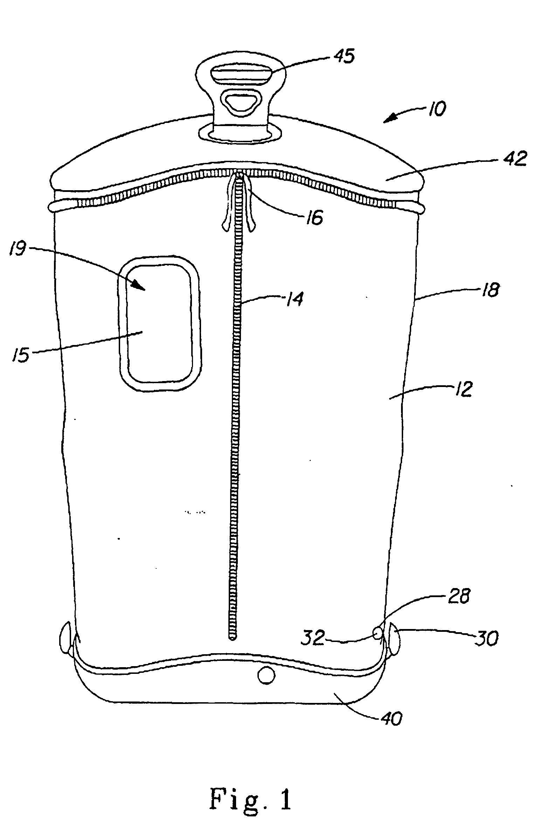

Referring now to Figure 1, which is a chematic representation of a fabric treatment apparatus 10 according to the present invention wherein the collapsible or expandable, preferably flexible walls 18 of container 12 are preferably made of a flexible material, which is preferably a lined fabric material. And more preferably the lining is a coating applied to the fabric by methods known to those skilled in the art such as transfer coating, direct coating. The fabric is preferably selected from the group consisting of cotton, polyester, nylon, rayon and mixtures thereof, and the lining is preferably selected from the group consisting of silicone, polyurethane, polyvinyl chloride and mixtures thereof. Collapsible or expandable walls 18 of container 12 define an interior void space 19, which is preferably supported by one of more rigid, yet collapsible frames 44. These frames can be separate from one another, or they can be a unitary structure. Interior void space 19 can be viewed via window 15 if collapsible or expandable walls 18 are made of an opaque material.

It is understood that while treatment apparatus 10 is shown in a rounded rectangular configuration, the present invention is not meant to be so limited. Other structural configurations are appropriate for this invention, for example, pyramid, spherical, hemispherical, two sided/garment bag and other configurations. Treatment apparatus 10 can be any appropriate size and shape to achieve the desired volumetric sizes disclosed herein. Fastener 16, which seals opening 14, can comprise virtually any known sealing device such as zippers, tape, ZIP LOCK® seals and hook and loop type fasteners, for example VELCRO®.

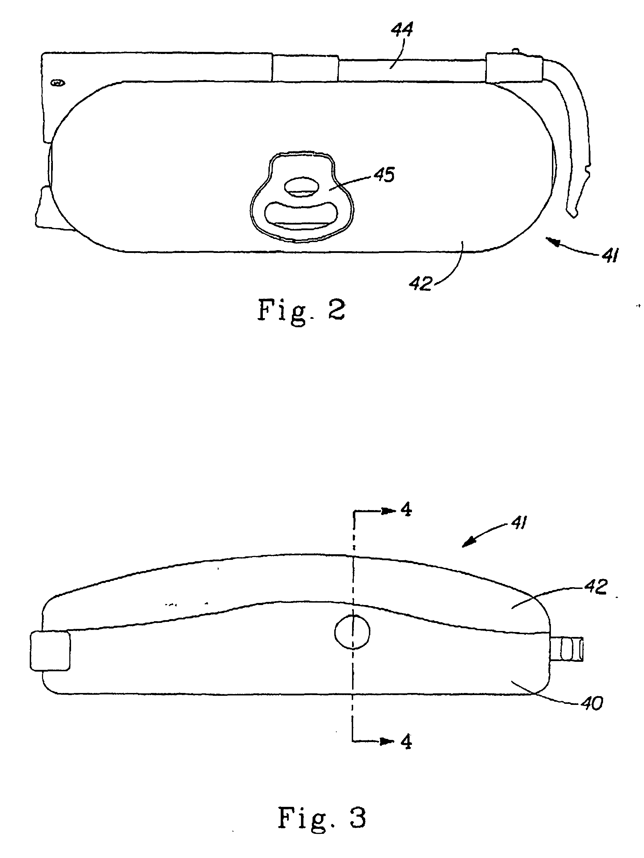

Turning now to Figures 2 and 3 which are the top and front views of the fabric treatment apparatus of Figure 1 after it has been collapsed. The containers of the present invention preferably comprise a rigid top portion 42 and a rigid bottom portion 40 that forms a receptacle 41 for the container when it is collapsed. If a frame is employed, for example 44, the rigid portions of the container can serve a support for the frame, or the frame and the rigid portion can be separate items that are not connected to one another. Preferably the frame or frames form a flexible, collapsible structure that when expanded forms a semi-rigid, three dimensional structure. Examples of collapsible structures are known, for example, in U.S. Patent No. 5,038,812, which issued on August 13, 1991, to Norman. In general, flexible, collapsible frames, such as those found in Norman, are formed from material that is relatively strong but nevertheless flexible enough to allow it to be collapsed. An exemplary frame material is flat spring steel having a rectangular cross section with dimensions of 1.6mm in width and 76mm in length. The frame or frames can be sewn, glued or otherwise attached to the interior or the exterior of the treatment bag. Likewise, the frame or frames can be free standing with the treatment bag material hanging loosely over, or being expanded by the frame.

As is discussed briefly above, the apparatuses of this invention are collapsible. That is, the container can be folded to substantially reduce its volume. More preferably, the container collapses into a receptacle that can be formed by the rigid portions of the container, or the receptacle can be a separate item. The receptacle need not be rigid, but can be any suitable storage unit for the collapsed container. Preferably the container comprises a handle that makes it easier to transport the collapsed container from one place to another. Even more preferably, the handle also serves as the exterior hanging means 45, which is used to hang the apparatus in use and can be used as a handle to carry receptacle 41 when apparatus 10 is collapsed.

To facilitate numerous cycles of collapsing and un-collapsing, the collapsible or expandable, preferably flexible material must be reasonably durable. By durable it is meant that the container should resist mechanical and chemical stress, that is the material should not swell, soften or develop cracks, holes, or other defects during its normal use. Likewise, if the container is constructed of a lined material, the lining should not deteriorate or exfoliate. In one preferred embodiment of this invention, the container is also thermally insulated with additional material, or even more preferably, the flexible material is a thermally insulating material. But as is discussed below in the Method description, there is a need for relatively quick "cool-down" of the bag which allows for condensation of the perfume on the fabrics. Thus, the bag should not be perfectly insulated.

The collapsible or expandable, preferably flexible, material should have a natural vapor permeability not higher than 3000, preferably, not higher than 2000, and more preferably not higher than 1000 grams of water/m²/day. Vapor permeability can be measured by a standardized test such as the ASTM E96 test, which will be known to those skilled in the art. The collapsible or expandable, preferably flexible, material can be essentially vapor impermeable, but it may be desirable for the container walls to have some limited permeabilty so the container can "breathe". Also, the collapsible or expandable, preferably flexible, material should be resistive to chemical corrosion, and ultra violet light. The various materials listed below as suitable cleaning and refreshment composition additives should not damage the container material over time. Likewise, the apparatuses of this invention may be used near a window wherein the sunlight might fade or otherwise damage the material. The container material should be selected to minimize this degradation due to natural sources. Suitable collapsible or expandable, preferably flexible, materials can be purchased from the Milliken Corp., in South Carolina, or the Sofinal Corp., in Belgium.

The containers of this invention can be formed from one sheet of collapsible or expandable, preferably flexible, material or from multiple sheets of material that are joined together in any appropriate manner. Those skilled in the art can contemplate many ways to join multiple sheets of material together to form a container. For example, the sheets can be sewn together, stapled, adhesively bonded, heat bonded, sonic bonded, or attached to one another by means that are known. The seams of container 10, if properly engineered, can form the container vent. By properly engineered, it is meant that the welds, stitches, bonds, staples, etc. of the container should be spaced so as to vent the desired amount of air during operation. Those skilled in the art will be able to determine the proper seam construct to achieve the desired venting without undue experimentation.

In addition to the at least one wall that defines an interior void space, the containers of this invention preferably comprise: a vent 28; a temperature controller 20 that is preferably active and is capable of changing and maintaining the air temperature within the interior void space 19 of container 12; a humidity provider 22 that is preferably passive and is capable of changing and maintaining the relative humidity of the air within the interior void space 19 of container 12; a heating element 24, which is capable of heating liquids to produce vapors and which can run dry to heat air; and an air circulation device 34, for example, a fan. Preferably, for the optimum deodorisation, it preferred to have air velocities around the garment between 0.05 to 10 m/s, more preferably between 0.1 and 5, most preferably between 0.5 and 2 m.s-1. Preferably, the active temperature controller, the passive humidity controller, the heating element, and the air circulation device are all within the interior void space 19 of container 12. Necessarily air circulation device 34 has an air inlet 36 and an air outlet 38, and it is preferred, that both air inlet 36 and air outlet 38 are located within interior void space 19 of container 12 so that at least a portion of the air within the interior void space 19 of container 12 is recirculated. Likewise, air outlet 38 is at least about 30 cm, preferably at least about 25 cm, and more preferably at least about 20 cm from vent 28 such that a portion of the air circulated within the interior void space 19 of container 12 is vented to the exterior of the container.

The vent is preferably selected from the group consisting of the natural permeability of the flexible material, seams created between sheets of the flexible material, seams between the container opening and the flexible material, a void space in the container material, and mixtures thereof. By void space in the container material it is meant that the vent can be any appropriately sized hole or opening. The filter 30 is a component of the apparatus. The filter 30 is preferably located at the top of the apparatus or at the bottom in either close proximity to the fan, thereby removing the need for a vent and the apparatus may then work in close system or under the cover plate in close proximity to the heating element. Preferably the filter 30 in close proximity, e.g. adjacent, the vent. Even more preferably the apparatus, most preferably the vent comprises a humidity sink, e.g. condensor 32 for condensing vapors before they are emitted from the container. The filter comprises an absorbent material, for example, activated carbon, to absorb fugitive chemicals, perfumes, malodorous compounds before they are emitted to the exterior of the container. Most preferably, the filter is a low pressure filter that has a low resistance to air. Typical of such filter are commercially available from AQF under the tradename CPS® or from MHB filtration. Preferably, part up to the total surface of the air circulation device, e.g. fan may be covered by the filter. If part of the air circulation device is covered, lost of the perfume through the filter is minimised whilst when the whole air circulation device is covered one can have the air circulation device automatically switched off upon the end of the cycle thereby enabling deposition of the perfume onto the garment. Condensors and filters are well known to those skilled in the appliance arts.

The apparatuses of this invention utilize hot vapors to clean and refresh fabric articles as described above. The vapors are typically created within the container by vaporizing a cleaning and refreshment composition, which comprises water and actives. The water and actives, that is, the "cleaning and refreshment composition", or "fabric treatment composition" (these two terms are used interchangeably throughout this description and are intended to mean the same thing), can be added to the container in any appropriate way. The composition can be poured into the bag, poured into a reservoir that feeds into the heating element/humidifier, canisters can be used to inject the composition, or an absorbent substrate saturated with the composition can be placed in the bag. Substrates and compositions suitable for use in the methods of this invention are described in greater detail below. It is understood that those skilled in the art will know of other methods of adding actives to the container and those methods are within the scope of this invention.

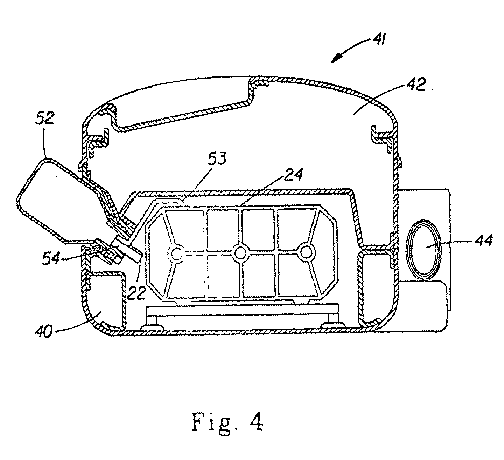

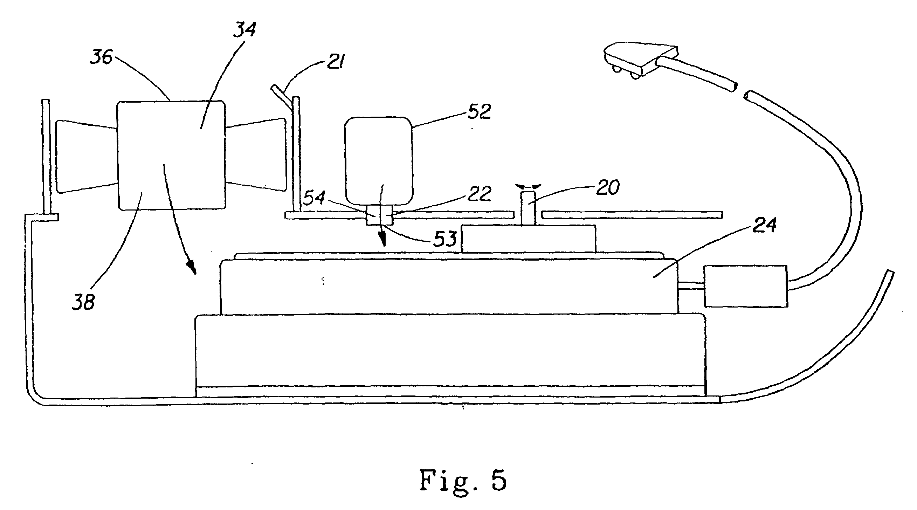

As discussed above, the apparatuses of this invention comprise a heating element and an air circulation device that work together to vaporize and distribute the cleaning and refreshment composition. By "work together" it is meant that the heating element is in fluid communication with the air outlet of the air circulation device such that as air is circulated within the interior void space of the container it contacts the heating element. Moreover, it is especially preferred that the heating element be in fluid communication with a fabric treatment composition that is vaporized by the heating element. One such mechanical system is shown in Figure 5, which is a schematic drawing of one possible arrangement of the mechanical components of the present invention. The fabric treatment composition is circulated throughout the interior void space of the container as air is circulated across the heating element carrying the vaporized fabric treatment composition. A cartridge 52 is shown in Figure 4, which is a cross sectional view of a fabric treatment apparatus of the present invention preferably comprise. The fabric treatment composition is contained within cartridge 52 having a cartridge outlet 53, wherein the cartridge outlet is in fluid communication with the heating element 24 via cartridge receiver 54..

The mechanical elements of apparatus 10 comprise, as a minimum, humidity provider 22, filter (30), heating element 24, and, as discussed above, air circulation device 34. Preferably, the apparatus also comprises a temperature controller. In a preferred aspect of this invention, heating element 24 serves to heat both the air and to vaporize the cleaning and refreshment composition. The vaporized cleaning and refreshment composition raises the humidity within the interior void space 19 of container 12. Humidity provider 22 is generally passive, that is it is preprogrammed to turn on and off based on the amount of vapor necessary to achieve the desired humidity. In contrast, temperature controller 20 is preferably active, that is the temperature is read with temperature probe 21 and this temperature is sent back to temperature controller 20. Based on the input from temperature probe 21, temperature controller 20 raises or lowers the temperature of heating element 24. Alternatively, heating element 24 can be turned on or off based on the input from controller 20. Each of these mechanical elements will be known to those skilled in the appliance arts, and the size and power of each element can be selected based on the volume of the container. Many manufacturers market these elements, such as, Etri in France, Blackmann in Austria, and IRCA in Italy.

While the apparatus described above arguably performs the function of a classic "steamer", it additionally heats and circulates dry air throughout the interior of the container. By this method both humidity and temperature can be controlled independently, which is not the case with prior steam generating apparatuses.

The vapor can be supplemented by a nebulizer, atomizer or the like device (not shown), which can be used to spray a fine mist of volatile and involatile cleaning and refreshment compositions. In addition to distributing a fine mist of the cleaning and refreshment composition throughout the interior of the container, the nebulizer can be used as the humidity provider as well. Preferably the nebulizer is an ultrasonic device, most preferably providing droplets size between 1-60 microns, most preferably between 1-40 microns. Nebulizers, atomizers and the like devices that are appropriate for use in the present invention are well known to those skilled in the art. A suitable device for use herein is a nebuliser which has an ultrasonic nozzle. Typical of such nebuliser is commercially available from Sono Tek Corporation, 2012 route 9W Building 3 in Milton New York 12547 under the tradename Acu Mist®. If used, it is preferred to have frequency set up to at least 60kHz, most preferably to at least 100 kHz so as to obtain droplets sizes below 60 microns, more preferably below 50 microns, most preferably below or equal to 40 microns. Still other examples of such devices can be purchased from the Omron, Health Care, GmbH, Germany, Flaem Nuove, S.p.A, Italy. Likewise, aerosol delivery systems, which are well known to the art, can be used to deliver the cleaning and refreshment compositions. More preferably, the nebuliser comprises protected cells. Indeed, a problem encountered with the use of cell containing nebuliser is their contamination from contact with the cleaning/refreshing composition, thereby causing build-up on the cell. As a result, the life time of the cell is shortened. It has now been found that protection of the cell, in particular by contacting the cell with a protective medium, e,g demineralised water, the latter being covered by a membrane, so that this system is closed, i.e. leak-free, solved this problem. Accordingly, the membrane is defined as providing the closing of the system but does not prevent the energy waves transmittal. Subsequently, the cleaning/refreshment composition is added on top of this system. As a result, the lifetime of the cells are greatly enhanced. One advantage of this system is that it can be run empty of cleaning/refreshment composition without the risk of destroying the cell and thus the nebuliser. Preferably, the membrane is a layer made of plastic film, and/or made of metal. Typical description of such apparatus can be found in a co-pending application BE 9900683 filed 14 October 1999 in the name of Brodsky SPRL. This finding is all the more surprising as previous attempts to solve this problem were by level detectors. However, this did not prevent the build-up from the cleaning/refreshment onto the cell.

In addition, it has also been found a means to improve the low output of the nebuliser. Indeed, another problem encountered with conventional nebuliser is that of the coalescence of the droplets. Indeed, as the droplets are emitted into the air, the higher they are the more they coalesce therefore giving bigger droplets and thus falling back into the basin of the nebuliser. The present invention solved this problem in a simple manner by the addition of a blowing means like a fan, which is preferably located on top of the nebuliser so as to provide a horizontal air flow and hence directing the flow of small droplets through a grid. Typical description of such apparatus can be found in a co-pending application BE 9900682 filed 14 October 1999 in the name of Brodsky SPRL.

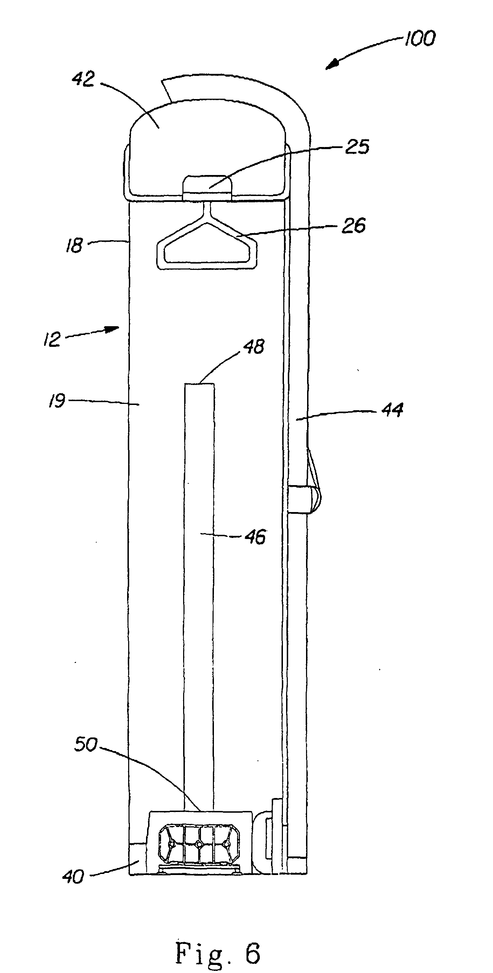

In one preferred aspect of this invention, as shown in Figure 6, which is a lengthwise cross sectional view of a fabric treatment apparatus 100 comprising a conduit 46 according to the present invention. More specifically, apparatus 100 comprises a conduit 46 having a conduit inlet 48 and a conduit outlet 50 wherein conduit outlet is in fluid communication with the air inlet 36 of the air circulation device 34 and wherein the air outlet 38 of air circulation device 34 is at least 25cm, preferably at least about 30cm and more preferably at least about 35cm from conduit inlet 48. Conduit 46 can be any appropriate device that air can flow through relatively unobstructed. For example a pipe, made of plastic or other materials can be used. An especially preferred conduit is a pipe having an interior diameter of from about 2 cm to about 10 cm.

Fabric articles can be suspended in the interior void space 19 of treatment apparatus 10 by any appropriate method. One such method is shown in Figure 6 wherein a bar 25 is provided to suspend hangars 26. The garments hung in treatment apparatus 10 can also be weighted or stretched to improve wrinkle reduction. Hanging weights and stretching devices will be known to those skilled in the art. Preferably, the garments to be treated are mechanically stretched after placing them into the container and before starting the process. This stretching or so-called tensioning of the garment helps the relaxation of wrinkles during the process. Preferred stretching systems include weighted as well as light weight compactable or retractible stretching systems, wherein the system comprises a tensioning device like a spring. The latter systems have the benefit of not adding extra weight to the cleaning and refreshing apparatus, along with the possibility of adjusting tensioning force and direction as required. Preferably, these systems are mounted inside the container at its bottom. One example of such as system is a rollerblind that is conventionally used as sunfilter for cars and commercially available from Halfords. This system is a rollerblind which can be extended or compacted by means of a roll-up spring mechanism. Only slight modification of this system are needed to adapt it to the tensioning of garment. One preferred adaptation involves attaching the housing of this system at the bottom of the apparatus and providing one or more clamp at the other side so that the clamping and thus the stretching or tensioning of the garment in the apparatus is obtained. The tension of the spring can also be adjusted to the desired stretching force for a given garment. The size of the clamp can vary so that more than one clamp are attached to this system. Still, another variation involves having only one clamp which run along or partly along the blind tensioning system located opposite the housing of the system.

Treatment apparatus 10 can be free standing with the support of a rigid frame 44, or it can be suspended by a hanging member 45 from a support means (not shown). If treatment apparatus 10 is suspended by hanging member 45 no frame is required although frames are generally preferred to control and maintain the shape and volume of interior void space 19. In a preferred embodiment of the present invention the container further comprises a rigid bottom portion 40, a rigid top portion 42 or both. These two rigid portions can be used to support the frame, house the mechanical elements of apparatus 10, and/or to serve as a housing for the collapsed container. Moreover, rigid bottom portion 40 and rigid top portion 42 can be designed to enhance the aesthetic characteristics of the apparatus, that is, there need not be any functionality to the rigid portions.

### Volume Refreshment Rate

The apparatuses of this invention must simultaneously clean and refresh fabrics with vaporous compositions, and vent out the malodorous vapors. It is understood that separating the desirable active vapors from the malodorous vapors would be a complex task. To simplify the apparatuses of this invention a Volume Refreshment Rate has been determined that optimizes the venting of malodorous compounds while minimizing the loss of active components from the cleaning and refreshment composition.

The Volume Refreshment Rate is defined as the frequency that the total volume of air within the interior void space of the container is replaced, expressed in units of seconds⁻¹. If the apparatus vents substantially lower than 0.0004s⁻¹ then venting becomes too weak, and deodorization performance deteriorates unless the cycle length is drastically increased. Theoretically, one volume refreshment per cycle could be enough to allow good deodorization. Supposing, for example, a cleaning and refreshment cycle takes 1 hour, of which the deodorization step would take approximately 40 minutes, this would mean a VR/s of 0.0004 s⁻¹. An exemplary Volume Refreshment Rate calculation is given in Example I below.

The Volume Refreshment Rate for the apparatuses of the present invention is between about 0.0004s⁻¹ and about 0.05s⁻¹, and preferably between about 0.001s⁻¹ and about 0.03s⁻¹.

### Method

To properly clean and refresh a fabric article, one must address many aspects of the article's appearance. Specifically, the fabric article should at least be substantially free of odor and wrinkles after a cleaning and refreshing operation. It is often preferred that the article be perfumed to give it a pleasant odor, and it should be free of localized stains. The methods of this invention require at least two steps designed toward deodorizing, dewrinkling and/or perfume deposition on a fabric article. Additionally, a manual spot removal process for removing localized stains is provided, but the spot removal process is conducted outside of the apparatus The conditions for each of these methods steps are described in greater detail below.

While the method steps of this invention can be carried out in any appropriate order, the deodorization step will be discussed first. Deodorization must be distinguished from odor-masking, which involves applying a pleasant scent to a fabric to mask, or cover up the odors on the fabric. Deodorization, as used herein, involves the actual removal or degradation of malodor causing chemicals. When the malodor causing constituents are removed or neutralized, the fabric article should have little or no residual odor. This step of the process can be carried out with ozone, which degrades odors, or with high temperatures and venting which removes the odor causing constituents.

The deodorization step, is described herein as the first step as a matter of convenience. It is understood that the deodorization and dewrinkling steps can be carried out in any order. If a perfume deposition step is employed, it necessarily should follow the deodorization step, so that the perfume is not stripped off of the fabric immediately after it is laid down.

Thus, when deodorization is the first step, the first temperature should be at least about 45°C, preferably at least about 60°C, and most preferably at least about 70°C and the first relative humidity should be least about 20%. At these relatively high temperatures, odor causing chemicals are stripped off of fabrics, and then preferably removed from the container via the vent. Even more preferably, the vent comprises a filter so that the odorous emanations do not enter the environment outside of the container. When the first temperature and first relative humidity are reached, the process time, that is, the first time, can be from about 2 minutes to about 20 minutes, preferably from about 5 minutes to about 15 minutes, and even more preferably from about 8 minutes to about 12 minutes.

The deodorization step described above can be supplemented, or even replaced by treating the fabric articles with ozone. The use of ozone to neutralize odors causing chemicals and to sanitize garments, for example, medical gowns, is well known to the art. Specifically see, published patent applications DE 24 33 909 and FR 2059 841. For purposes of the methods disclosed herein, ozone can be introduced into the container from any appropriate source, such as an ultraviolet lamp or even a high voltage source. One or more ozone sources can be used and they can be placed in any convenient place in, or adjacent the exterior of the container. The ozone source must be sized according to the volume of the container with consideration for the surface area of the fabric articles being cleaned and refreshed. An alternative way to produce ozone for deodorization is the use of high voltage. For example, a wire can be placed in the container and approximately about 10,000 volts passed across the wire. This generally serves the same purpose as the UV lamp generating ozone. Those skilled in the art will know what type and size of equipment to use for a given container.

The second step of the present invention is directed to dewrinkling, which requires relatively high temperature and relative humidity. Good air circulation that agitates the fabrics and evenly distributes the active ingredients is beneficial to the dewrinkling step, but not necessary. For the second step, i.e. the dewrinkling step, the second temperature should be greater than "T" as defined by the equation: T = 60 - (0.17 * RH₂), wherein RH₂ is the second relative humidity in percent. RH₂ is of at least 50%, preferably of at least 75%, more preferably of at least about 85%, most preferably at least about 90%. Preferably, the second temperature is less than about 90°C, more preferably less than about 80°C, and most preferably less than about 70°C. When the second temperature and second relative humidity are reached, the process time, that is, the second time, can be from about 2 minutes to about 20 minutes, preferably from about 5 minutes to about 15 minutes, and even more preferably from about 8 minutes to about 12 minutes.

Finally, there is preferably a third step which involves a gradual cool down of the interior void space. As the temperature decreases, the amount of vapor that the air can retain in the air decreases, and when the air becomes saturated the vapors begin to condense. Naturally, vapors will condense on the fabric articles on the inside of the bag, and as these articles dry, the active ingredients, such as perfume, remain behind. As discussed briefly above, the methods steps of this invention are designed to deliver actives without undue waste and without saturating the fabrics to the point where they need additional drying. Preferably, during the third step in the process the temperature within the interior void space decreases to a third temperature wherein the third temperature is less than about 45°C, preferably less than about 40°C, and more preferably less than about 35°C. This third step can last for a third period of time, which can be from about 2 minutes to about 20 minutes, preferably from about 3 minutes to about 10 minutes, and even more preferably from about 3 minutes to about 5 minutes.

As discussed in greater detail below, the vapor inside the container is preferably a cleaning and refreshment composition. The cleaning refreshment composition can be added to the container directly, via a sheet/substrate, in a cartridge or any other means that will be known to those skilled in the art. Preferably, the cleaning and refreshment composition is in a cartridge that is introduced into the interior void space of the container and the cleaning and refreshment composition is released from the cartridge into the interior void space of the container.

### Cleaning/Refreshment Composition

The cleaning/refreshment composition preferably comprises water and optionally a member selected from the group consisting of surfactants, perfumes, preservatives, bleaches, auxiliary cleaning agents, shrinkage reducing compositions, organic solvents and mixtures thereof. The preferred organic solvents are glycol ethers, specifically, methoxy propoxy propanol, ethoxy propoxy propanol, propoxy propoxy propanol, butoxy propoxy propanol, butoxy propanol, ethanol, isopropanol, wrinkle removing agents, in-wear anti-wrinkling agents, semi-durable press agents, odor absorbing agents, volatile silicones and mixtures thereof. Fabric shrinkage reducing compositions that are suitable for use in the present invention are selected from the group consisting of ethylene glycol, all isomers of propanediol, butanediol, pentanediol, hexanediol and mixtures thereof. More preferably, the fabric shrinkage reducing compositions are selected from the group consisting of neopentyl glycol, polyethylene glycol, 1,2-propanediol, 1,3-butanediol, 1-octanol and mixtures thereof. The surfactant is preferably a nonionic surfactant, such as an ethoxylated alcohol or ethoxylated alkyl phenol, and is present at up to about 2%, by weight of the cleaning/refreshment composition. Preferred auxiliary cleaning agents include cyclodextrins and dewrinkling agents, such as silicone containing compounds. Especially preferred anti-wrinkling agents include volatile silicones, some of which can be purchased from the Dow Corning Corporation. One such volatile silicone is D5 cyclomethicone decamephyl cyclopenta siloxane. Typical fabric cleaning/refreshment compositions herein can comprise at least about 80%, by weight, water, preferably at least about 90%, and more preferably at least about 95% water.

The Examples below give specific ranges for the individual components of preferred cleaning/refreshment compositions for use herein. A more detailed description of the individual components of the cleaning/refreshment compositions, that is, the organic solvents, surfactants, perfumes, preservatives, bleaches and auxiliary cleaning agents can be found in U.S. Patent No. 5,789,368, which issued on August 4, 1998 to You et al. Additionally, cleaning/refreshment compositions are described in co-pending U.S. Patent Application No. 08/789,171, which was filed on January 24, 1997, in the name of Trinh et al. And shrinkage reducing compositions for use in this invention can be found in co-pending U.S. Provisional Application No. 60/097,596, entitled "Cleaning Compositions that Reduce Fabric Shrinkage", which was filed by Strang and Siklosi, on August, 24, 1998.

### Spot Cleaning Composition

The user of the present process can be provided with various spot cleaning compositions to use in the optional pre-spotting procedure of this invention. These compositions are used to remove localized stains from the fabrics being treated, either before or after the cleaning and refreshing process defined herein. Necessarily, the spot cleaning composition must be compatible with the fabric being treated. That is, no meaningful amount of dye should be removed from the fabric during the spot treatment and the spot cleaning composition should leave no visible stains on the fabric. Therefore, in a preferred aspect of this invention there are provided spot cleaning compositions which are substantially free of materials that leave visible residues on the treated fabrics. This necessarily means that the preferred compositions are formulated to contain the highest level of volatile materials possible, preferably water, typically about 95%, preferably about 97.7%, and surfactant at levels of about 0.1% to about 0.7%. A preferred spot cleaning composition will also contain a cleaning solvent such as butoxy propoxy propanol (BPP) at a low, but effective, level, typically about 1% to about 4%, preferably about 2%.

Preferred spot cleaning methods and compositions are described in U.S. Patent No. 5,789,368, to You et al.. Additionally, spot cleaning methods and compositions are described in U.S. Patent No. 5,630,847, which issued on May 20, 1997, to Roetker.

### Treatment Member

In one embodiment, a treatment member is provided to assist in removing localized stains from fabrics. In a preferred aspect of this invention, the spot cleaning composition is provided in a dispenser, such as a bottle, and the dispenser has a distal tip that can serve as the treatment member. Additionally, the treatment member can comprise an absorbent base material which can be, for example, a natural or synthetic sponge, an absorbent cellulosic sheet or pad, or the like. In contact with and extending outward from this base material can be multiple protrusions. Specific examples of treatment members can be found in U.S. Patent No. 5,789,368, to You et al.

### Absorbent Stain Receiving Article

An absorbent stain receiving article, sometimes referred to herein as a stain receiver, can optionally be used in the optional pre-spotting operations herein. Such stain receivers can be any absorbent material which imbibes the liquid composition used in the pre-spotting operation. Disposable paper towels, cloth towels such as BOUNTY™ brand towels, clean rags, etc., can be used. However, in a preferred mode the stain receiver is designed specifically to "wick" or "draw" the liquid compositions away from the stained area. One preferred type of stain receiver consists of a nonwoven pad, such as a thermally bonded air laid fabric ("TBAL"). Another highly preferred type of stain receiver for use herein comprises polymeric foam, wherein the polymeric foam comprises a polymerized water-in-oil emulsion, sometimes referred to as "poly-HIPE". The manufacture of polymeric foam is very extensively described in the patent literature; see, for example: U.S. Patent No. 5,260,345 to DesMarais, Stone, Thompson, Young, LaVon and Dyer, issued November 9, 1993; U.S. Patent No. 5,550,167 to DesMarais, issued August 27, 1996, and U.S. 5,650,222 to DesMarais et al., issued July 22, 1997. Typical conditions for forming the polymeric foams of the present invention are described in co-pending U.S. Patent Application Serial No. 09/042,418, filed March 13, 1998 by T. A. DesMarais, et al., titled "Absorbent Materials for Distributing Aqueous Liquids". Additional disclosure of conditions for forming the polymeric foams for use in the present invention are described in co-pending U.S. Provisional Patent Application Serial No. 60/077,955, filed March 13, 1998 by T. A. DesMarais, et al., titled "Abrasion Resistant Polymeric Foam And Stain Receivers Made Therefrom".

The various stain receivers described herein, and described in the references incorporated herein by reference, preferably comprise a liquid impermeable backsheet. The backsheet can be made of, for example, a thin layer of polypropylene, polyethylene and the like. The backsheet provides protection for the surface that the stain receiver rests on from the spot cleaning composition. For example, spot cleaning processes are typically performed on a hard surface, such as a table top. The stain receiver is placed on the table and the fabric to be treated in placed on the stain receiver. Spot cleaning composition is applied to the stained area of the fabric and then drawn into the stain receiver. But in the absence of a back sheet, the spot cleaning composition can leak onto the table top, possibly causing damage thereto.

The following Example II further illustrates the invention, but is not intended to be limiting thereof.

### EXAMPLE I

An apparatus having an interior void space with a volume of 0.25m³ is used for calculating the Volume Refreshment Rate. The vent comprises the natural ventilation through the seams of the container. The air within the container is heated to 75°C by circulating air over a heated metal fin heat exchanger using a fan that circulates air at 96 liters/s. The relative humidity within the container is raised to about 85% by slowly dispensing 125g of water, at a rate of 12.5 g/minute on to the hot metal fins of the heat exchanger. By this method the vaporized steam is immediately mixed with the hot air and then circulated throughout the interior void space of the container. The air that is vented from the container has 85% relative humidity and is 75°C.

The vent has a surface area of 0.002 m² (the vent comprises a 5 cm diameter hole). The surface area of the vent is divided by 2 because approximately half of the vent draws air into the bag at approximately the same rate that air is emitted from the bag. In operation air exits the container through the top half of the vent. Thus, only half of the vent hole actually vents air and vapor, thus, the effective surface area of the vent is 0.001 m². An empirical measurement of the air flow from the vent hole determines that the air and vapor exits the container at a velocity of 0.75 m/s. Thus, the volumetric flow rate of air and vapor leaving the bag is 0.00075 m3/s (0.001 m² * 0.75 m/s). This flow rate is divided by 0.25 m3 (bag volume) = 0.003s⁻¹. For a typical cycle time of 20 minutes (1200 seconds) there are 3.6 refreshments / cycle.

### EXAMPLE II

Two extra-large men's jackets that have been exposed to cigarette smoke and wrinkled using standardized methods, are placed on clothes hangers. These jackets are then hung on the inside of a plastic bag that has two co-planer flat ends (the top and bottom) with the side walls being cylindrical and slightly outwardly bowed near the center. For illustration purposes only, the bag can be thought of as shaped like an egg shell with the top and bottom cut off. The container has a door for accessing the interior, and the door is closed with a zipper. A circle opening near the bottom of the bag serves as the vent and the vent remains open at all times during this process. There is an activated carbon filter in the opening that comprises the vent.

On the interior of the bag is a fan, a heating element, a thermostat and a reservoir that is in fluid communication with the heating element. Approximately 100 to 150 mls of a composition comprising approximately 99% water and 1% perfume, by weight, is poured into the reservoir and the door is closed.

An exterior "on/off" switch is turned on to begin the fabric refreshment process. The switch is connected to a programmable microprocessor that controls the multi-step process. First, the temperature is raised to about 70°C with a relative humidity of about 50%. This is accomplished by running the fan and the heating element, with little or no cleaning and refreshment composition in contact with the heating element. This first step lasts for about 10 to 15 minutes. For the second step, the temperature is reduced to about 50°C and the relative humidity is raised to greater than about 75%. This is accomplished through the introduction of the vaporized cleaning and refreshment composition. The fan continues to run during this second step, which lasts for about 7 to 9 minutes.

Finally, with the fan running the heating element is turned off and the interior of the bag cools naturally to about 45°C in less than about 10 minutes. The fan is turned off automatically, and an indicator light signals that the process is complete. The jackets are removed and they are substantially wrinkle free, deodorized and ready to wear.

## Claims

1. An apparatus (10) for treating a fabric article comprising:
a) a collapsible or expandable container (12) that is made from a material that defines an interior void space having an open volume of between about 0.75 m³ and about 0.05 m³, preferably between about 0.6 m³ and about 0.1 m³, between about 0.5 m³ and about 0.2 m³, and further comprising an opening;
b) a humidity provider (22);
c) a heating element (24);
d) a vent (28);
e) an air circulation device (34); and
wherein the container can be collapsed to at least about 50%, preferably at least about 40%, and more preferably at least about 25% of its open volume, **characterized in that** the vent (28) and the air circulation device (34) are dimensioned in order to have a volume refreshment rate of between 0,0004 s⁻¹ and 0,05 s⁻¹, preferably between 0,001 s⁻¹ and 0,035 s⁻¹.

2. The apparatus (10) according to Claim 1, wherein the humidity provider (22) is a humidity controller capable of changing and maintaining the relative humidity of the air within the interior void space of the container (12).

3. The apparatus (10) according to Claim 1, wherein the apparatus further comprises a temperature controller (20) capable of changing and maintaining the air temperature within the interior void space of the container (12).

4. The apparatus (10) according to Claim 1, wherein the apparatus further comprises a humidity sink.

5. The apparatus (10) according to Claim I, wherein the humidity sink is a condenser (32).

6. The apparatus (10) according to claim 1, wherein the material is a flexible material, preferably a fabric material that can withstand temperatures of about 100°C with essentially no increase in its vapor permeability.

7. The apparatus (10) according to claim 1, wherein the apparatus weighs less than about 15 Kg, preferably less than about 10 Kg, and more preferably less than about 8 Kg.

8. The apparatus (10) according to claim 1, wherein the humidity provider (22), the heating element (24), the air circulation device (34), the optional temperature controller (20), the optional humidity controller, the optional humidity sink and the filter are all within the interior void space of the container (12).

9. The apparatus (10) according to claim 1, wherein the air circulation device (34) has an air inlet (36) and an air outlet (38), and both the air inlet (36) and the air outlet (38) are located within the interior void space (19) of the container (12) so that at least a portion of the air within the void space (19) of the container (12) is recirculated.

10. The apparatus (10) according to claim 9, wherein the heating element (24) is in fluid communication with the air outlet (38) of the air circulation device (34) such that as air is circulated within the interior void space (19) of the container (12) it contacts the heating element (24).

11. The apparatus (10) according to claim 10, wherein the heating element (24) is in fluid communication with a fabric treatment composition that is vaporized by the heating element.

12. The apparatus (10) according to claim 11, wherein the fabric treatment composition is circulated throughout the interior void space (19) of the container (12) as air is circulated across the heating element (24) carrying the vaporized fabric treatment composition.

13. The apparatus (10) according to Claim 1, wherein the air circulation device (34) provides air velocities around the garment between 0.05 to 10 m.s⁻¹, more preferably between 0.1 and 5m.s⁻¹, most preferably between 0.5 and 2 m.s⁻¹.

14. The apparatus (10) according to claim 11, wherein the fabric treatment composition comprises water and a perfume.

15. The apparatus (10) according to claim 1, further comprising an ozone source within the interior void space (19) of the container (12).

16. The apparatus (10) according to claim 15, wherein the ozone source is an ultraviolet lamp or a high voltage source.

17. The apparatus (10) according to claim 11, wherein the fabric treatment composition is contained within a cartridge having a cartridge outlet, wherein the cartridge outlet is in fluid communication with the heating element.

18. The apparatus (10) according to claim 1, wherein the interior void space (19) is supported by a collapsible frame.

19. The apparatus (10) according to claim 1, wherein the fabric is a lined material selected from the group consisting of cotton, polyester, nylon, rayon and mixtures thereof, and the lining is selected from the group consisting of silicone, polyurethane, polyvinyl chloride and mixtures thereof.

20. The apparatus (19) according to claim 1, wherein the humidity provider (22) is a nebulizer, which is preferably ultrasonic.

21. The apparatus (10) according to Claim 20, wherein the nebuliser is an ultrasonic nozzle.

22. The apparatus (10) according to Claim 20, wherein the nebuliser comprises one or more cells which are protected from the cleaning/refreshment composition.

23. The apparatus (10) according to Claim 22, wherein the cell protection is achieved by a membrane made of plastic film and/or metal.

24. The apparatus (10) according to Claim 20, wherein the nebuliser comprises a fan.

25. The apparatus (10) according to claim 17, wherein the cleaning refreshing composition in the cartridge comprises water and optionally a member selected from the group consisting of surfactants, perfumes, preservatives, bleaches, auxiliary cleaning agents, shrinkage reducing compositions, organic solvents and mixtures thereof, preferably the organic solvents are glycol ethers, more preferably the organic solvents are selected from the group consisting of methoxy propoxy propanol, ethoxy propoxy propanol, propoxy propoxy propanol, butoxy propoxy propanol, butoxy propanol, ethanol, isopropanol, wrinkle removing agents, in-wear anti-wrinkling agents, semi-durable press agents, odor absorbing agents, volatile silicones and mixtures thereof.

26. The apparatus (10) according to claim 9, further comprising a conduit (46) having a conduit inlet (48) and a conduit outlet (50) wherein the conduit outlet (50) is in fluid communication with the air inlet (36) of the air circulation device (34) and wherein the air outlet (38) of the air circulation device (34) is at least 25cm, preferably at least about 30cm and more preferably at least about 35cm from the conduit inlet (48).

27. The apparatus (10) according to Claim 1, wherein the filter (30) is in close proximity to the air circulation device (34).

28. The apparatus (10) according to claim 1, wherein the vent (28) further comprises a filter.

29. The apparatus (10) according to claim 1, wherein the vent (28) further comprises a condenser.

30. The apparatus (10) according to claim 1, wherein the vent (28) is selected from the group consisting of the natural vapor permeability of the bag material, seams created between sheets of the flexible material, seams between the container opening and the flexible material, a void space in the container material, and mixtures thereof.

31. The apparatus (10) according to claim 1, wherein a portion of the container (12) is rigid, and the rigid portion forms a receptacle for the container when it is collapsed.

32. An apparatus according to claim 1, which further comprises
a hangar (26) for suspending at least one fabric article within the interior void space (19) of the container (12).

33. The apparatus (10) according to claim 1, wherein the material is a flexible material, preferably has a natural vapor permeability not higher than 3000, preferably, not higher than 2000, and more preferably not higher than 1000 grams of water/m²/day.

34. The apparatus (10) according to Claim 1, wherein the apparatus further comprises one or more stretching devices.

## Patentansprüche

1. Vorrichtung zur Behandlung eines Textilgegenstands mit
a) einem zusammenfaltbaren oder ausziehbaren Behälter (12), der aus einem Material gefertigt ist, das einen Innenhohlraum im Bereich von etwa 0,75m³ bis etwa 0,05m³, vorzugsweise im Bereich von etwa 0,6m³ bis etwa 0,1m³ und besser noch im Bereich von etwa 0,5m³ bis etwa 0,2m³ definiert, und ferner eine Öffnung aufweist,
b) einem Befeuchter (22),
c) einem Heizelement (24),
d) einer Lüftung (28),
e) einer Luftzirkulationsvorrichtung (34),
wobei der Behälter auf mindestens etwa 50%, vorzugsweise mindestens etwa 40% und besser noch auf mindestens etwa 25% seines offenen Volumens zusammengefaltet werden kann,
**dadurch gekennzeichnet,**
**dass** die Lüftung (28) und die Luftzirkulationsvorrichtung (34) derart bemessen sind, dass sie eine Volumenerneurungsrate im Bereich von 0,0004s⁻¹ bis 0,05s⁻¹, vorzugsweise im Bereich von 0,001s⁻¹ bis 0,03s⁻¹, aufweisen.

2. Vorrichtung (10) nach Anspruch 1,
**dadurch gekennzeichnet,**
**dass** der Befeuchter (22) eine Feuchtigkeitssteuervorrichtung ist, die die relative Luftfeuchtigkeit im Innenhohlraum des Behälters (12) ändern und aufrecht erhalten kann.

3. Vorrichtung (10) nach Anspruch 1,
**dadurch gekennzeichnet,**
**dass** sie femer eine Temperatursteuervorrichtung (20) aufweist, die die Lufttemperatur im Innenhohlraum des Behälters (12) ändern und aufrecht erhalten kann.

4. Vorrichtung (10) nach Anspruch 1,
**dadurch gekennzeichnet,**
**dass** sie ferner ein Feuchtigkeisabsenkungsmittel aufweist.

5. Vorrichtung (10) nach Anspruch 1,
**dadurch gekennzeichnet,**
**dass** das Feuchtigkeitsabsenkungsmittel ein Kondensator (32) ist.

6. Vorrichtung (10) nach Anspruch 1,
**dadurch gekennzeichnet,**
**dass** das Material ein flexibles Material, vorzugsweise ein Textilmaterial, ist, das Temperaturen von etwa 100°C ohne wesentlichen Anstieg seiner Dampfdurchlässigkeit widerstehen kann.

7. Vorrichtung (10) nach Anspruch 1,
***dadurch gekennzeichnet,***
**dass** sie weniger als etwa 15kg, vorzugsweise weniger als etwa 10kg und noch besser weniger als etwa 8kg, wiegt.

8. Vorrichtung (10) nach Anspruch 1,
**dadurch gekennzeichnet,**
**dass** der Befeuchter (22), das Heizelement (24), die Luftzirkulationsvorrichtung (34), die wahlfreie Temperatursteuervorrichtung (20), die wahlfreie Feuchtigkeitssteuervorrichtung, das wahlfreie Feuchtigkeitsabsenkungsmittel und das Filter alle im Innenhohlraum des Behälters (12) untergebracht sind.

9. Vorrichtung (10) nach Anspruch 1,
**dadurch gekennzeichnet,**
**dass** die Luftzirkulationsvorrichtung (34) einen Lufteinlass (36) und einen Luftauslass (38) aufweist, die beide im Innenhohlraum (19) des Behälters (12) derart angeordnet sind, dass mindestens ein Teil der Luft im Innenhohlraum (19) des Behälters (12) wieder zurückgeführt wird.

10. Vorrichtung (10) nach Anspruch 9,
**dadurch gekennzeichnet,**
**dass** das Heizelement (24) mit dem Luftauslass (38) der Luftzirkulationsvorrichtung (34) derart in Fluidverbindung steht, dass bei der Zirkulation der Luft im Innenhohlraum (19) des Behälters (12) die Luft das Heizelement (24) kontaktiert.

11. Vorrichtung (10) nach Anspruch 10,
**dadurch gekennzeichnet,**
**dass** das Heizelement (24) in Fluidverbindung mit einer Textilbehandlungszusammensetzung steht, die durch das Heizelement verdampft wird.

12. Vorrichtung (10) nach Anspruch 11,
**dadurch gekennzeichnet,**
**dass** die Textilbehandlungszusammensetzung im Kreislauf durch den Innenhohlraum (19) de Behälters (12) getrieben wird, wenn Luft über das Heizelement (24) geführt wird, das die verdampfte Textilbehandlungszusammensetzung trägt.

13. Vorrichtung (10) nach Anspruch 1,
**dadurch gekennzeichnet,**
**dass** die Luftzirkulationsvorrichtung (34) für Luftgeschwindigkeiten um die Bekleidung im Bereich von 0,05 bis 10m·s⁻¹, vorzugsweise im Bereich von 0,1 bis 5m·s⁻¹ und noch besser im Bereich von 0,5 bis 2m·s⁻¹, sorgt.

14. Vorrichtung (10) nach Anspruch 11,
**dadurch gekennzeichnet,**
**dass** die Textilbehandlungszusammensetzung Wasser und Parfum aufweist.

15. Vorrichtung (10) nach Anspruch 1,
**dadurch gekennzeichnet,**
**dass** sie ferner eine Ozonquelle im Innenhohlraum (19) des Behälters (12) aufweist.

16. Vorrichtung (10) nach Anspruch 15,
**dadurch gekennzeichnet,**
**dass** die Ozonquelle eine Ultraviolettlampe oder eine Hochspannungsquelle ist.

17. Vorrichtung (10) nach Anspruch 11,
**dadurch gekennzeichnet,**
**dass** die Textilbehandlungszusammensetzung in einer Patrone mit einem Patronenauslass enthalten ist, der mit dem Heizelement in Fluidverbindung steht.

18. Vorrichtung (10) nach Anspruch 1,
**dadurch gekennzeichnet,**
**dass** der Innenhohlraum (19) durch einen zusammenfaltbaren Rahmen gestützt wird.

19. Vorrichtung (10) nach Anspruch 1,
**dadurch gekennzeichnet,**
**dass** das Textil ein gefüttertes Material ist, das aus einer Gruppe ausgewählt ist, die Wolle, Polyester, Nylon, Rayon und Mischungen daraus umfasst, und dass das Futter aus einer Gruppe ausgewählt ist, die Silikon, Polyurethan, Polyvinylchlorid und Mischungen daraus umfasst.

20. Vorrichtung (10) nach Anspruch 1,
**dadurch gekennzeichnet,**
**dass** der Befeuchter (22) ein Zerstäuber ist, der vorzugsweise mit Ultraschall arbeitet.

21. Vorrichtung (10) nach Anspruch 20,
**dadurch gekennzeichnet,**
**dass** der Zerstäuber eine Ultraschalldüse ist.

22. Vorrichtung (10) nach Anspruch 20,
**dadurch gekennzeichnet,**
**dass** der Zerstäuber eine oder mehrere Zellen aufweist, die vor der Reinigungs- und Auffrischungszusammensetzung geschützt sind.

23. Vorrichtung (10) nach Anspruch 22,
***dadurch gekennzeichnet,***
**dass** der Zellschutz durch eine Membran erreicht wird, die aus einem Kunststofffilm und/oder aus Metall besteht.

24. Vorrichtung (10) nach Anspruch 20,
**dadurch gekennzeichnet,**
**dass** der Zerstäuber einen Lüfter umfasst.

25. Vorrichtung (10) nach Anspruch 17,
**dadurch gekennzeichnet,**
**dass** die Reinigungs- und Auffrischungszusammensetzung in der Patrone Wasser und wahlfrei ein Glied aufweist, das aus einer Gruppe ausgewählt ist, die Tenside, Parfums, Konservierungsmittel, Bleichmittel, Zusatzreinigungsmittel, einlaufvermindemde Zusammensetzungen, organische Lösungsmittel und Mischungen daraus umfasst, wobei die organischen Lösungsmittel vorzugsweise Glykolether oder noch besser aus einer Gruppe ausgewählt sind, die Methoxy-Propoxy-Propanol, Ethoxy-Propoxy-Propanol, Propoxy-Propoxy-Propanol, Butoxy-Propoxy-Propanol, Butoxy-Propanol, Ethanol, Isopropanol, entknitterungsbeseitigende Mittel, verschleißbare Entknitterungsmittel, halbhaltbare Bügelfaltenmittel, Geruchabsorptionsmittel, flüchtige Silikone und Mischungen daraus umfasst.

26. Vorrichtung (10) nach Anspruch 9,
**dadurch gekennzeichnet,**
**dass** sie ferner eine Rohrleitung (46) mit einem Rohrleitungseingang (48) und einem Rohrleitungsausgang 50 aufweist, der mit dem Lufteinlass (36) der Luftzirkulationsvorrichtung (34) in Fluidverbindung steht, wobei der Luftauslass (38) der Luftzirkulationsvorrichtung (34) mindestens 25cm, vorzugsweise mindestens etwa 30cm und noch besser mindestens etwa 35cm vom Rohrleitungseingang (48) entfernt liegt.

27. Vorrichtung (10) nach Anspruch 1,
**dadurch gekennzeichnet,**
**dass** das Filter (30) in unmittelbarer Nähe der Luftzirkulationsvorrichtung (3) angeordnet ist.

28. Vorrichtung (10) nach Anspruch 1,
**dadurch gekennzeichnet,**
**dass** die Lüfung (28) ferner ein Filter aufweist.

29. Vorrichtung (10) nach Anspruch 1,
**dadurch gekennzeichnet,**
**dass** die Lüfung (28) ferner einen Kondensator aufweist.

30. Vorrichtung (10) nach Anspruch 1,
**dadurch gekennzeichnet,**
**dass** die Lüftung (28) aus einer Gruppe ausgewählt ist, die aus der natürlichen Dampfdurchlässigkeit des Beutelmaterials, den zwischen den Bahnen des flexiblen Materials geschaffenen Säumen, den Säumen zwischen der Behälteröffnung und dem flexiblen Material, einem Hohlraum im Behältermaterial und Mischungen daraus besteht.

31. Vorrichtung (10) nach Anspruch 1,
**dadurch gekennzeichnet,**
**dass** ein Teil des Behälters (12) fest ist und ein Aufnahmegefäß für den Behälter bildet, wenn dieser zusammengefaltet ist.

32. Vorrichtung (10) nach Anspruch 1,
**dadurch gekennzeichnet,**
**dass** sie femer einen Aufhänger (26) zum Aufhängen mindestens eines Textilgegenstands im Innenhohlraum (19) des Behälters (12) aufweist.

33. Vorrichtung (10) nach Anspruch 1,
**dadurch gekennzeichnet,**
**dass** das Material ein flexibles Material ist, das vorzugsweise eine natürliche Dampfdurchlässigkeit von nicht mehr als 3000, besser nicht mehr als 2000 und noch besser nicht mehr als 1000g/m²/Tag aufweist.

34. Vorrichtung (10) nach Anspruch 1,
**dadurch gekennzeichnet,**
**dass** sie ferner ein oder mehrere Reckvorrichtungen aufweist.

## Revendications

1. Appareil (10) pour traiter un article de tissu comprenant :
a) un conteneur pliable ou expansible (12) qui est fait en un matériau qui définit un espace vide interne ayant un volume ouvert entre environ 0,75 m³ et environ 0,05 m³, de préférence entre environ 0,6 m³ et environ 0,1 m³, entre environ 0,5 m³ et environ 0,2 m³, et comprenant en outre une ouverture ;
b) un humidificateur (22) ;
c) un élément chauffant (24) ;
d) une ventilation (28) ;
e) un dispositif de circulation d'air (34) ; et
dans lequel le conteneur peut être plié à au moins environ 50 %, de préférence au moins environ 40 %, et plus préférablement au moins environ 25 % de son volume ouvert, **caractérisé en ce que** la ventilation (28) et le dispositif de circulation d'air (34) sont dimensionnés de manière à avoir un taux de rafraîchissement volumique entre 0,0004 s⁻¹ et 0,05 s⁻¹, de préférence 0,001 s⁻¹ et 0,03 s⁻¹.

2. Appareil (10) selon la revendication 1, dans lequel l'humidificateur (22) est un contrôleur d'humidité capable de changer et de maintenir l'humidité relative de l'air dans l'espace vide interne du conteneur (12).

3. Appareil (10) selon la revendication 1, dans lequel l'appareil comprend en outre un contrôleur de température (20) capable de changer et de maintenir la température de l'air dans l'espace vide interne du conteneur (12).

4. Appareil (10) selon la revendication 1, dans lequel l'appareil comprend en outre un bac d'humidité.

5. Appareil (10) selon la revendication 1, dans lequel le bac d'humidité est un condenseur (32).

6. Appareil (10) selon la revendication 1, dans lequel le matériau est un matériau flexible, de préférence un matériau de tissu qui peut supporter des températures d'environ 100°C avec essentiellement pas d'augmentation de sa perméabilité à la vapeur.

7. Appareil (10) selon la revendication 1, dans lequel l'appareil pèse moins d'environ 15 kg, de préférence moins d'environ 10 kg, et plus préférablement moins d'environ 8 kg.

8. Appareil (10) selon la revendication 1, dans lequel l'humidificateur (22), l'élément chauffant (24), le dispositif de circulation d'air (34), le contrôleur de température optionnel (20), le contrôleur d'humidité optionnel, le bac d'humidité optionnel et le filtre sont tous dans l'espace vide inteme du conteneur (12).

9. Appareil (10) selon la revendication 1, dans lequel le dispositif de circulation d'air (34) présente une entrée d'air (36), une sortie d'air (38, et qu'à la fois l'entrée d'air (36) et la sortie d'air (38) sont situées dans l'espace vide inteme (19) du conteneur (12) de manière qu'au moins une partie de l'air dans l'espace vide (19) du conteneur (12) soit recyclé.

10. Appareil (10) selon la revendication 9, dans lequel l'élément chauffant (24) est en communication fluide avec la sortie d'air (38) du dispositif de circulation d'air (34) de manière que quand l'air circule dans l'espace vide inteme (19) du conteneur (12) il soit en contact avec l'élément chauffant (24).

11. Appareil (10) selon la revendication 10, dans lequel l'élément chauffant (24) est en communication fluide avec une composition de traitement de tissu qui est vaporisée par l'élément chauffant.

12. Appareil (10) selon la revendication 11, dans lequel la composition de traitement de tissu circule dans tout l'espace vide interne (19) du conteneur (12) quand l'air circule à travers l'élément chauffant (24) transportant la composition de traitement de tissu vaporisée.

13. Appareil (10) selon la revendication 1, dans lequel le dispositif de circulation d'air (34) fournit des vitesses d'air autour du vêtement entre 0,05 et 10 m.s⁻¹, plus préférablement entre 0,1 et 5 m.s⁻¹, encore plus préférablement entre 0,5 et 2 m.s⁻¹.

14. Appareil (10) selon la revendication 11, dans lequel la composition de traitement de tissu comprend de l'eau et un parfum.

15. Appareil (10) selon la revendication 1, comprenant en outre une source d'ozone dans l'espace vide inteme (19) du conteneur (12).

16. Appareil (10) selon la revendication 15, dans lequel la source d'ozone est une lampe à ultraviolets ou une source à haute tension.

17. Appareil (10) selon la revendication 11, dans lequel la composition de traitement de tissu est contenue dans une cartouche ayant une sortie de cartouche, où la sortie de cartouche est en communication fluide avec l'élément chauffant.

18. Appareil (10) selon la revendication 1, dans lequel l'espace vide interne (19) est supporté par un cadre pliable.

19. Appareil (10) selon la revendication 1, dans lequel le tissu est un matériau doublé choisi dans le groupe constitué par le coton, le polyester, le nylon, la rayonne et leurs mélanges, et la doublure est choisie dans le groupe constitué par la silicone, le polyuréthane, le polychlorure de vinyle et leurs mélanges.

20. Appareil (10) selon la revendication 1, dans lequel l'humidificateur (22) est un nébuliseur, qui est de préférence à ultrasons.

21. Appareil (10) selon la revendication 20, dans lequel le nébuliseur est une buse à ultrasons.

22. Appareil (10) selon la revendication 20, dans lequel le nébuliseur comprend une ou plusieurs cellules qui sont protégées de la composition de nettoyage/rafraîchissement.

23. Appareil (10) selon la revendication 22, dans lequel la protection de la cellule est obtenue par une membrane faite d'un film plastique et/ou d'un métal.

24. Appareil (10) selon la revendication 20, dans lequel le nébuliseur comprend un ventilateur.

25. Appareil (10) selon la revendication 17, dans lequel la composition de nettoyage rafraîchissement dans la cartouche comprend de l'eau et en option un élément choisi dans le groupe constitué par des tensioactifs, des parfums, des conservateurs, des agents de blanchiment, des agents de nettoyage auxiliaires, des compositions réduisant le rétrécissement, des solvants organiques et leurs mélanges, de préférence les solvants organiques sont des éthers de glycol, plus préférablement les solvants organiques sont choisis dans le groupe constitué par le méthoxypropoxypropanol, l'éthoxypropoxypropanol, le propoxypropoxypropanol, le butoxypropoxypropanol, le butoxypropanol, l'éthanol, l'isopropanol, des agents éliminant les plis, des agents anti-froissement lors du port, des agents de pressage semi-permanents, des agents absorbant les odeurs, des silicones volatiles et leurs mélanges.

26. Appareil (10) selon la revendication 9, comprenant en outre une conduite (46) ayant une entrée de conduite (48) et une sortie de conduite (50) dans laquelle la sortie de conduite (50) est en communication fluide avec l'entrée d'air (36) du dispositif de circulation d'air (34) et dans lequel la sortie d'air (38) du dispositif de circulation d'air (34) est à au moins 25 cm, de préférence au moins environ 30 cm et plus préférablement au moins environ 35 cm de l'entrée de conduite (48).

27. Appareil (10) selon la revendication 1, dans lequel le filtre (30) est au voisinage proche du dispositif de circulation d 'air (34).

28. Appareil (10) selon la revendication 1, dans lequel la ventilation (28) comprend en outre un filtre.

29. Appareil (10) selon la revendication 1, dans lequel la ventilation (28) comprend en outre un condenseur.

30. Appareil (10) selon la revendication 1, dans lequel la ventilation (28) est choisie dans le groupe constitué par la perméabilité naturelle à la vapeur du matériau de sac, des coutures créées entre les feuilles du matériau flexible, des coutures entre l'ouverture du conteneur et le matériau flexible, un espace vide dans le matériau du conteneur, et leurs mélanges.

31. Appareil (10) selon la revendication 1, dans lequel une partie du conteneur (12) est rigide, et la partie rigide forme un réceptacle pour le conteneur quand il est plié.

32. Appareil (10) selon la revendication 1, qui comprend en outre un cintre (26) pour suspendre au moins un article de tissu dans l'espace vide interne (19) du conteneur (12).

33. Appareil (10) selon la revendication 1, dans lequel le matériau est un matériau flexible, de préférence présente une perméabilité naturelle à la vapeur non supérieure à 3000, de préférence non supérieure à 2000, et plus préférablement non supérieure à 1000 grammes d'eau/m²/jour.

34. Appareil (10) selon la revendication 1, dans lequel l'appareil comprend en outre un ou plusieurs dispositifs d'étirage.
